Europäisches Patentamt

European Patent Office

Office européen des brevets

(11) Publication number: **0 260 012**
**A1**

## (12) EUROPEAN PATENT APPLICATION

(21) Application number: 87307499.1

(22) Date of filing: 25.08.87

(51) Int. Cl.⁴: **C12N 15/00 , C07H 21/04 , C12N 5/00 , C07K 15/14**

(30) Priority: 02.09.86 US 885901

(43) Date of publication of application:
16.03.88 Bulletin 88/11

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Ellis, Ronald W.
1407 Edgevale Road
Overbrook Hills Pennsylvania 19151(US)
Inventor: Kieff, Elliott
5744 South Kimbark
Chicago Illinois 60637(US)

(74) Representative: Hesketh, Alan, Dr.
European Patent Department Merck & Co.,
Inc. Terlings Park Eastwick Road
Harlow Essex, CM20 2QR(GB)

(54) Promoter for expression of heterologous proteins in mammalian cells.

(57) The promoter of gpI of varicella-zoster virus (VZV), a later promoter during VZV infections, has been isolated and demonstrated to be active in directing the expression of a heterologous gene in mammalian cells in the absence of any other element of the VZV geome. This promoter is useful for the expression of recombinant DNA-derived proteins in mammalian cells.

Figure 1

# PROMOTER FOR EXPRESSION OF HETEROLOGOUS PROTEINS IN MAMMALIAN CELLS

## BACKGROUND OF THE INVENTION

Mammalian cells grown in vitro are versatile host cells for the expression of recombinant DNA-derived proteins. Among the most widely used host cells are rodent fibroblast lines, e.g., Ltk⁻, NIH3T3 and Chinese hamster ovary, and African green monkey kidney (AGMK) lines, e.g., vero. One widely used application of these cells has been the production of proteins of direct human therapeutic value, including vaccines, enzymes and hormones. In this regard, the biology of the cell substrate is very important, since concern has been expressed over the use of proteins expressed from tumorigenic cell lines. Among the many cell lines which have been tested for tumorgenicity, the vero line of AGMK cells has been shown to be non-tumorigenic when tested at lower passage levels. Therefore, such vero cells are a desirable cell substrate for the expression of heterologous proteins by recombinant DNA technology.

A number of potent promoters for mammalian cells have been described. Some of these, such as the early promoter of simian virus 40 (SV40), are active in a wide range of cell lines. Others, such as the immediate early promoter of cytomegalovirus (CMVIE), are not active in all cell lines tested. Since the number of available promoters is limited in scope, the identification of a novel promoter is useful. Furthermore, due to the difficulty of regulating promoter activity in mammalian cells in general, a promoter of highest potency is not always desirable. If high levels of a toxic protein are constitutively expressed in a cell line, non-expressor cells can be selected rapidly such that productive scale-up of a cell line becomes difficult. In addition, in light of the desirability of vero cells as described above, a promoter that is functional in vero cells is valuable for the expression of proteins of therapeutic interest.

Chickenpox is caused by varicella-zoster virus (VZV), a member of the herpesvirus family. As with other herpesviruses, the expression of VZV genes is temporally regulated into three groups, immediate early, early, and late. Among the late genes are those encoding the glycoproteins of the virion. The most abundant VZV glycoprotein is gpI, whose mRNA is abundant during the late phase of VZV infection. This suggests that the VZV gpI promoter is potent during the course of a VZV infection. Late promoters of herpesviruses are known to require the expression of other genes earlier in the course of infection in order to be activated. Therefore, it is not obvious that a late promoter, such as gpI, would be capable of functioning as a promoter for mammalian cells in the absence of other VZV genes, i.e., in the absence of "transactivation."

Epstein-Barr virus (EBV) is the etiologic agent of infectious mononucleosis and is implicated in the development of Burkitt's lymphoma and undifferentiated nasopharyngeal carcinoma. The EB virion has three major surface glycoproteins: gp350 (350,000 dalton glycoprotein), gp220 and gp85. The gp350 and gp220 polypeptides are the products of a single viral gene. These two glycoproteins are capable of eliciting the production of antibodies capable of neutralizing EBV infectivity in vitro. Therefore, the gp350 gene and its products are useful for the preparation of a vaccine to EBV-induced disease through the use of recombinant DNA techniques. It would be desirable to express the gp350 gene in vero cells, since glycosylation is important to the immunogenicity of gp350 and since monkey cells should confer a pattern of glycosylation upon gp350 which is most similar to that found in the course of the natural viral infection in humans.

## OBJECTS OF THE INVENTION;

It is an object of the present invention to provide the VZV gpI promoter as a novel promoter for the expression of recombinant-derived proteins in mammalian cells. It is a further object to incorporate this promoter into a vector to make it generally useful as a mammalian expression vector. Yet another object is to provide a method for expressing EBV gp350 in vero cells. These and other objects of the invention will be apparent from the following description.

## SUMMARY OF THE INVENTION:

The VZV gpI promoter has been utilized to express a heterologous gene in mammalian cells in the absence of any other elements of the VZV genome. This promoter is useful for the expression of recombinant-derived proteins in mammalian cells.

## Brief Description of the Drawings:

Figure I is a schematic diagram illustrating the construction of pAM8.

Figure 2 is a schematic diagram illustrating the construction of pMAI02,
which was used in the construction of pAM8.

Figure 3 is a schematic diagram illustrating the construction of the pAM8/3 line of vero cells, which expresses EBV gp350.

## DETAILED DESCRITPION OF THE INVENTION :

The present invention is directed to the use of the promoter of the VZV gpI gene for the expression of heterologous genes in mammalian cells. In particular, this promoter is active in vero cells, a non-tumorigenic line of AGMK cells useful in the production of proteins of therapeutic interest for humans. Aside from their biology, vero cells are useful for the expression of glycoproteins. Owing to their primate origin, vero cells would be expected to glycosylate heterologous proteins to a form more closely related to that found in humans than would non-primate cells, such as rodent cells. In some glycoproteins, the pattern of glycosylation is extremely important to biological activity or immunogenicity.

The VZV gpI promoter is isolated and utilized as follows:

The SacI G fragment of VZV, which circumscribes the complete gpI gene, is cloned into a modified pBR322 which contains a SacI site instead of a BamHI site. The resulting plasmid (PVSGO-I2) is digested with AvaI and the 3.7 kbp fragment is purified by preparative agarose gel electrophoresis. This fragment is digested with EcoRV and the 0.9 kbp fragment is purified by preparative agarose gel electrophoresis. Four oligonucleotides are synthesized, hybridized and ligated to form a 55 base pair (bp) AvaI-XbaI linker. This linker is ligated to the 0.9 kbp fragment, and the resulting 0.9 kbp fragment (containing the gpI promoter) is isolated by preparative agarose gel electrophoresis. The structural gene for EBV gp350 and gp220 is identical and is wholly contained within the BamHI L genomic DNA fragment in plasmid B68'. This plasmid is digested with BamHI. The 5.0 kbp fragment is purified by preparative agarose gel electrophoresis, digested with BanI, flush-ended with T4 DNA polymerase and digested with HindIII. The resulting 0.3 kbp fragment is isolated by preparative agarose gel electrophoresis and cloned into the HincII and HindIII sites of pUCI9, thus generating pMAIOI. The B68' plasmid is digested with XhoI, and the resulting 4.2 kbp fragment is isolated by preparative agarose gel electrophoresis and cloned into the unique XhoI site of pMAIOI, thus generating pMAI02. The 0.9 kbp fragment (containing the gpI promoter) is cloned into the Xba I site and flush-ended KpnI site of pMAI02, thus generating pAM8. This plasmid contains the VZV gpI promoter and RNA cap site, the complete EBV gp350 coding sequence, transcriptional termination sequences and a polyA addition site.

The pAM8 plasmid is co-transfected into vero cells with pSV2-gpt, which contains a dominant selectable marker. Transformants are isolated in selective growth medium and screened for the production of EBV gp350 by means of specific monoclonal antibodies. Lysates prepared from positive clones are resolved by sodium dodecylsulfate-polyacrylamide gel electrophoresis (SDS-PAGE), immunoblotted onto nitrocellulose and screened for the presence of gp350/gp220 with polyclonal monospecific rabbit antibodies to gp350. By this criterion, positive clones are identified.

The gpI promoter is contained within the reconstructed 0.9 kbp EcoRV-XbaI fragment described above. A fragment smaller than 0.9 kbp would be sufficient to function as a promoter in mammalian cells. Furthermore, there is variation in DNA sequence among different strains of VZV, and some nucleotides within the promoter region could be deleted or mutated without affecting the functionality of the promoter. Therefore, it will be obvious to those skilled in the art that the 0.9 kbp fragment is identifiable as the gpI promoter of VZV and can undergo some variation in sequence and size while still maintaining functionality as a promoter.

The EBV gp350 gene is but one example of a gene whose expression can be directed by the VZV gpI promoter. Often, but not always, the most useful of such genes would be those encoding proteins of direct therapeutic value in humans. The EBV gp350 gene has no intrinsic qualities which make it unique relative to other genes for expression by this promoter. Thus it will be obvious to those skilled in the art that the principle of inserting the EBV gp350 gene adjacent to the VZV gpI promoter for the purpose of expressing gp350/gp220 extends to any other protein-coding region.

Owing to their non-tumorigenic nature, vero cells are useful for the expression of proteins of direct therapeutic value to humans. However, many other cell lines have been used for the expression of proteins, and the VZV gpI promoter can be expected to be active in many of these other cell lines. Therefore, it will be obvious to those skilled in the art that the utility of the VZV gpI promoter for the expression of heterologous genes extends to other mammalian cell lines, including, but not limited to HeLa, NIH3T3, MK2, Chinese hamster ovary, and Ltk⁻.

The VZV gpI promoter is useful for the expression of EBV gp350 in vero cells. This result demonstrates that vero cells are capable of expressing this glycoprotein, which is known to exert toxic effects upon its host cells. Therefore, it will be obvious to those skilled in the art that the principle of utilizing the VZV gpI promoter for the expression of EBV gp350 extends to other promoters which are functional in vero cells.

The following examples illustrate the present invention without, however, limiting the same thereto. The disclosure of each reference mentioned in the following examples is incorporated by reference.

EXAMPLE I

Construction of an Expression Cassette Containing the VZV gpI Promoter :

The VZV gpI glycoprotein represents one of the major structural glycoproteins contained in the VZV virion [Ellis et al., J. Virology, 53:81 (1985)]; the transcription rate directed by the VZV gpI promoter is relatively high. The SacI G fragment of VZV, whose complete DNA sequence has been reported [Davison et al., J. Gen. Virol. 64:1181 (1983)] and which circumscribes the complete gpI gene, was cloned into the SacI site of a modified pBR322-(pRES), created by digesting pBR322 with BamHI, flush-ending with the Klenow fragment of DNA polymerase I, ligating with an octamer oligonucleotide containing the restriction site for SacI (Collaborative Research), and then digesting with SacI. The resulting plasmid (pVSGO-12 Figure IA) was digested with AvaI, and the 3.7 kbp fragment was purified by preparative agarose gel electrophoresis. This fragment was digested with EcoRV, and the resulting 0.9 kbp fragment was purified by preparative agarose gel electrophoresis. This fragment contains VZV gpI promoter sequences excluding 0.05 kbp immediately 5′ to the ATG translational initiation codon of VZV gpI. Four oligonucleotides with the following sequences were synthesized:

```
#1    5′-TCGGGCGAATTGCGTGGTTTTAAG
#2    CGCTTAACGCACCAAAATTCACTGAT
- 5′

#3    5′-TGACTATATTCCGAGGGTCGCCTG-
TAT
#4    ATAAGGCTCCCAGCGGACATAGATC -
5′.
```

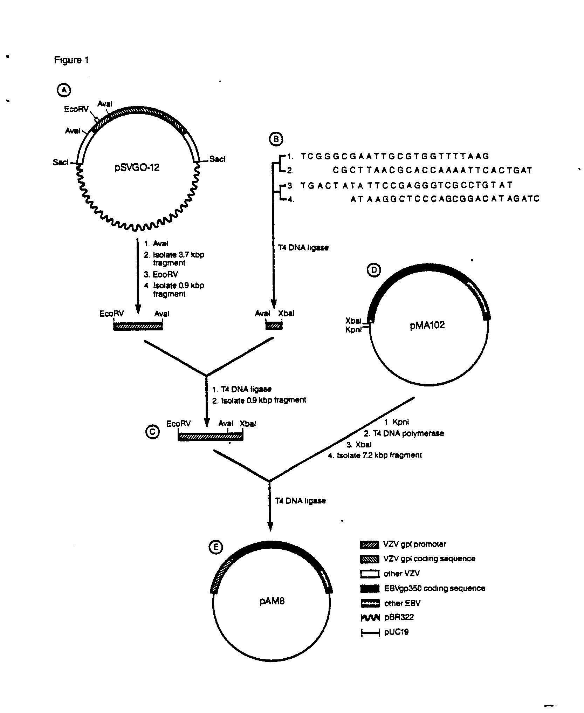

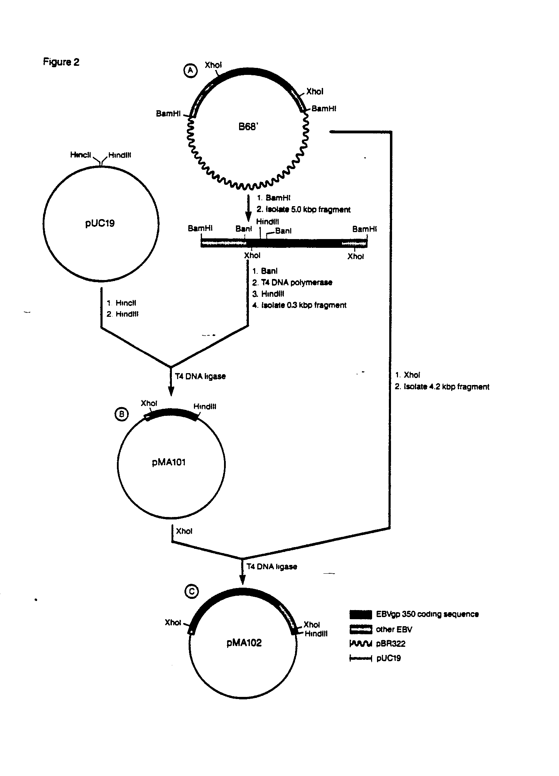

Oligonucleotides #1 and #2 were hybridized in the presence of 0.9M NaCl, 0.IM sodium citrate, pH 7.4, 0.0IM EDTA by heating the solution to 65°C and gradually cooling to 23°C. Oligonucleotides #3 and #4 also were hybridized under the same conditions. These two oligonucleotides then were ligated to each other, generating a 55 bp linker containing AvaI and Xba I 5′ overhangs which represents the VZV gpI promoter sequence immediately 5′ to the coding sequence of VZV gpI (Figure IB). This 55 bp linker was ligated to the 0.9 kbp fragment of pVSGO-12, thus generating another 0.9 kbp fragment which was purified by preparative agarose gel electrophoresis (Figure IC). The structural gene for EBV gp350 and gp220 is identical and is wholly-contained within the BamHI L genomic DNA fragment in plasmid B68′ [Hummel et al., J. Virology, 49:413 (1984)]. The B68′ plasmid was digested Bam HI, and the 5.0 kbp fragment was purified by preparative agarose gel electrophoresis (Figure 2A). This fragment was digested with with Ban I, flush-ended with T4 DNA polymerase and digested with Hind III. The resulting 0.3 kbp fragment was purified by preparative agarose gel electrophoresis and cloned into the HincII and Hind III sites of pUCI9 [Yanisch-Perran et al., Gene, 33:103 (1985)], thus generating pMAI0I (Figure 2B). The B68′ plasmid was digested with XhoI, and the resulting 4.2 kbp fragment was purified by preparative agarose gel electrophoresis and cloned into the unique XhoI site of pMAI0I, generating pMAI02 (Figure 2C) which contains the entire structural gene for EBV gp350. The plasmid pMAI02 (Figure ID) was digested with KpnI, flush-ended with T4 DNA polymerase, and digested with XbaI. The resulting 7.2 kbp fragment was purified by preparative agarose gel electrophoresis and ligated with the 0.9 kbp EcoV-Xba I fragment containing the VZV gpI promoter, thus generating pAM8 (Figure IE). This plasmid was used to generate a vero cell line expressing EBV gp350 as described in Example 2 and contains the following salient features: 1) the VZV gpI promoter and RNA cap site; 2) the complete protein coding sequence for EBV gp350; and 3) sequence for transcriptional termination and PolyA addition.

EXAMPLE 2

Generation of a Stable Vero Cell Line Expressing EBV gp350:

The plasmid pSV2-gpt contains the E. coli xgprt gene (5-phospho-α-ribose-I-diphosphate: xanthine phosphoribosyltransferase) transcribed under the direction of the SV40 early promoter (Mulligan et al., Proc. Natl Acad. Sci. U.S.A. 78:2072 (1981)].

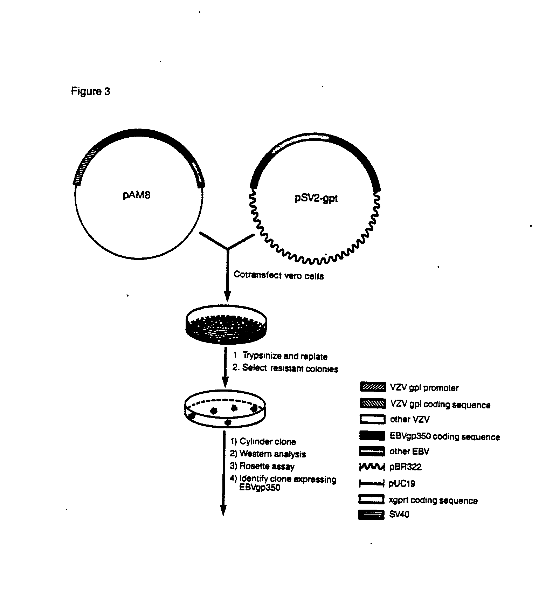

This gene can be used as a dominant selectable marker in the transfection of mammalian cells. I0-20 μg of pAM8 (Example I) and 5 μg of pSV2-gpt were diluted to 250 μl in water, and 250 μl of 0.5 M CaCl2 were added. The DNA mixture was extracted with an equal volume of chloroform:isoamyl alcohol (24:I), and the aqueous phase was added dropwise to 500 μl of 280 mM NaCl, 50 mM Hepes, I.4 mM Na2HP04' PH 7.I while bubbling air through the mixture. A precipitate was allowed to form for 60 min. at 23°C. The precipitate was added to I0 ml of growth media [Dulbecco's Modified Eagle's Medium (DMEM) plus I0% fetal calf serum] covering a 90% confluent monolayer of vero cells and incubated 4-8 hr in a 37°C C02 incubator (Figure 3). Then the media were replaced with fresh media and incubated an additional 24 hr. The transfected cells were trypsinized and replated in selective media, i.e., growth media containing 0.025% (w/v) xanthine, 0.00I5% (w/v) hypoxanthine, 0.015% (w/v) L-glutamine, 0.001% (w/v) thymidine, 0.0002% (w/v) aminopterin, 0.0025% (w/v) mycophenolic acid as described by Mulligan et al. (ibid.). Five to ten days later, colonies of cells resistant to the selective media were cylinder-cloned and grown to confluence in fresh plates.

In order to assay individual clones for the expression of EBV gp350, lysates were prepared, electrophoresed in 5.5% SDS-polyacrylamide gels, i@munoblotted onto nitrocellulose and sequentially incubated with polyclonal rabbit anti-EBV serum [North et al. Int. J. of Cancer 26:23I (I980)] and [$^{125}$I] Protein A. Clone pAM8/3 was found by this assay to express both gp350 and gp220. Another assay used to rapidly assay expressor clones is described as follows: The growth media were replaced by fresh growth media containing I0 μg/ml of a monoclonal antibody specific for EBV gp350 [Cl.4, Thorley-Lawson et al., Proc. Nat. Acad. Sci. U.S.A. 77:5307 (I980)] and incubated for I hour at 37°C. The cells then were washed 3 times with DMEM and incubated for I hour at 37°C in the presence of growth media containing human red blood cells to which rabbit anti-mouse immunoglobulin had been coupled according to the following procedure: Human red blood cells (type AB positive) were washed 5 times at 23°C in I50 mM NaCl. Two ml of rabbit anti-mouse IgG (I mg/ml) were added with gentle vortexing to I ml of washed and packed red blood cells. Two ml of 0.033% (w/v) chromic chloride, pH 5.0, were added dropwise to the red blood cells with constant mixing. The cells then were rocked for 7 min. at 23°C, washed 5 times at 4°C in I50mM NaCl, washed I time at 4@C in Hank's Balanced Salt Solution (HBSS) and resuspended in 50 ml HBSS for use at a I:I0 dilution. After I hour of incubation, the transfected cells were washed 3 more times with DMEM and screened visually for rosettes of red blood cells; such rosettes are indicative of cells expressing gp350. By this criterion, pAM8/3 was found to be a stable producer of EBV gp350.

## Claims

I. The following DNA sequence flanking the 5' end of the coding sequence of the gpI gene of varicella-zoster virus as a promoter for mammalian cells:

```
ATCAACAG AGCCGGATGC TGGTGTAATG
TTGAAAATTA   CCAAACCGGG   AATAAATGAT
GCTGGTGTGT   ATGTACTTCT   TGTTCGGTTA
GACCATAGCA   GATCCACCGA   TGGTTTCATT
CTTGGTGTAA   ATGTATATAC   AGCGGGCTCG
CATCACAACA   TTCACGGGGT   TATCTACACT
TCTCCGTCTC   TACAGAATGG   ATATTCTACA
AGAGCCCTTT   TTCAACAAGC   TCGTTTGTGT
GATTTACCCG   CGACACCCAA   AGGGTCCGGT
ACCTCCCTGT   TTCAACATAT   GCTTGATCTT
CGTGCCGGTA   AATCGTTAGA   GGATAACCCT
TGGTTACATG AGGACGTTGT TACGACAGAA ACTAAGTCCG   TTGTTAAGGA   GGGGATAGAA
AATCACGTAT   ATCCAACGGA   TATGTCCACG
TTACCCGAAA   AGTCCCTTAA   TGATCCTCCA
GAAAATCTAC   TTATAATTAT   TCCTATAGTA
GCGTCTGTCA TGATCCTCAC CGCCATCCTT ATTGTTATTG   TAATAAGCGT   TAAGCGACGT
AGAATTAAAA   AACATCCAAT   TTATCGCCCA
AATACAAAAA   CAAGAAGGGG   CATACAAAAT
GCGACACCAG   AATCCGATGT   GATGTTGGAG
GCCGCCATTG   CACAACTAGC   AACGATTCGC
GAAGAATCCC   CCCCACATTC   CGTTGTAAAC
CCGTTTGTTA AATAGAACTA ATTATCCCGG AT-
TTTATATT   AAATAAACTA   TATGCGTTTT   ATT-
TAGCGTT TTGATTACGC GTTGTGATAT GAGGG-
GAAGG   ATTAAGAATC   TCCTAACTAT   AAGT-
TAACAC   GCCCACATTT   GGGCGGGGAT
GTTTTATGAA GCCTTAAAGG CCGAGCTGGT ATACACGAGA   GCAGTCCATG   GTTTTAGACC
TCGGGCGAAT   TGCGTGGTTT   TAAGTGACTA
TATTCCGAGG GTCGCCTGTA
```

2. A recombinant plasmid containing at least a portion of the DNA sequence specified in Claim I.

3. A recombinant plasmid according to Claim 2 which contains a protein coding sequence adjacent to the promoter sequence.

4. A recombinant plasmid according to Claim 3 wherein the protein coding sequence is at least a portion of gp350/gp220 of Epstein-Barr virus.

5. A mammalian cell containing a recombinant plasmid according to Claim 2.

6. A mammalian cell containing a recombinant plasmid according to Claim 3.

7. A mammalian cell containing a recombinant plasmid according to Claim 4.

8. A vero cell containing a recombinant plasmid according to Claim 2.

9. A vero cell containing a recombinant plasmid according to Claim 3.

I0. A vero cell containing a recombinant plasmid according to Claim 4.

II. A recombinant DNA-derived protein purified from a mammalian cell according to Claim 6.

I2. A recombinant DNA-derived protein purified from a mammalian cell according to Claim 7.

I3. A recombinant DNA-derived protein purified from a vero cell according to Claim 9.

I4. A recombinant DNA-derived protein purified from a vero cell according to Claim I0.

I5. The gp350/gp220 products, or portions thereof, from Epstein-Barr virus derived from a mammalian cell according to Claim 7.

I6. The gp350/gp220 products, or portions thereof, from Epstein-Barr virus derived from a vero cell according to Claim I0.

I7. The gp350/gp220 products, or portions thereof, from Epstein-Barr virus derived from a vero cell.

Figure 1

(A) pSVGO-12

EcoRV
Aval
Aval
SacI
SacI

1. Aval
2. Isolate 3.7 kbp fragment
3. EcoRV
4. Isolate 0.9 kbp fragment

(B)
1. TCGGGCGAATTGCGTGGTTTTAAG
2.    CGCTTAACGCACCAAAATTCACTGAT
3. TGACTATATTCCGAGGGTCGCCTGTAT
4.    ATAAGGCTCCCAGCGGACATAGATC

T4 DNA ligase

EcoRV    Aval          Aval    Xbal

(D) pMA102

Xbal
Kpnl

1. T4 DNA ligase
2. Isolate 0.9 kbp fragment

(C) EcoRV    Aval   Xbal

1. Kpnl
2. T4 DNA polymerase
3. Xbal
4. Isolate 7.2 kbp fragment

T4 DNA ligase

(E) pAM8

VZV gpl promoter
VZV gpl coding sequence
other VZV
EBVgp350 coding sequence
other EBV
pBR322
pUC19

**Figure 2**

(A) XhoI

XhoI

BamHI

BamHI

**B68'**

HincII  HindIII

**pUC19**

1. BamHI
2. Isolate 5.0 kbp fragment

HindIII

BamHI    BanI    BanI                    BamHI

XhoI                          XhoI

1. BanI
2. T4 DNA polymerase
3. HindIII
4. Isolate 0.3 kbp fragment

1. HincII
2. HindIII

T4 DNA ligase

1. XhoI
2. Isolate 4.2 kbp fragment

(B) XhoI      HindIII

**pMA101**

XhoI

T4 DNA ligase

(C)

XhoI                    XhoI
                        HindIII
**pMA102**

EBVgp 350 coding sequence
other EBV
pBR322
pUC19

Figure 3

pAM8

pSV2-gpt

Cotransfect vero cells

1. Trypsinize and replate
2. Select resistant colonies

1) Cylinder clone
2) Western analysis
3) Rosette assay
4) Identify clone expressing
   EBVgp350

VZV gpl promoter

VZV gpl coding sequence

other VZV

EBVgp350 coding sequence

other EBV

pBR322

pUC19

xgprt coding sequence

SV40

| | DOCUMENTS CONSIDERED TO BE RELEVANT | | EP 87307499.1 | |
|---|---|---|---|---|
| Category | Citation of document with indication, where appropriate, of relevant passages | Relevant to claim | CLASSIFICATION OF THE APPLICATION (Int. Cl.4) | |
| D,A | JOURNAL OF VIROLOGY, vol. 53, no. 1, January 1985, Washington, DC.<br><br>R.W. ELLIS et al. "Use of Bacterial Expression Vector to Map the Varicella-Zoster Virus Major Glycoprotein Gene, gC"<br>pages 81-88<br><br>  * Totality *<br><br>-- | 1 | C 12 N 15/00<br>C 07 H 21/04<br>C 12 N 5/00<br>C 07 K 15/14 | |
| P,A | EP - A2 - 0 210 931 (MERCK & CO. INC.)<br><br>  * Page 2, lines 1-10; claim 1 *<br><br>-- | 1 | | |
| P,A | EP - A2 - 0 192 902 (MERCK & CO. INC.)<br><br>  * Page 2, lines 1-9; claims 1-3 *<br><br>-- | 1 | TECHNICAL FIELDS SEARCHED (Int. Cl.4) | |
| D,A | PROCEEDINGS OF THE NATIONAL ACADEMY OF SCIENCES OF THE UNITED STATES OF AMERICA, vol. 77, no. 9, September 1980, Baltimore, USA<br><br>D.A. THORLEY-LAWSON et al. "Monoclonal antibodies against the major glycoprotein (gp 350/220) of Epstein-Barr virus neutralize infectivity"<br>pages 5307-5311<br><br>  * Totality *<br><br>-- | 15-17 | C 12 N<br>C 07 H<br>C 07 K | |

The present search report has been drawn up for all claims

| Place of search | Date of completion of the search | Examiner |
|---|---|---|
| VIENNA | 09-12-1987 | WOLF |

| | **DOCUMENTS CONSIDERED TO BE RELEVANT** | | EP 87307499.1 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2 - 0 151 079 (THE UNIVERSITY OF CHICAGO) <br> * Pages 2,3; claim 2 * <br> -- | 15-17 | |
| A | EP - A1 - 0 173 254 (WOLF) <br> * Claims 1-3; fig. 17 * <br> ---- | 15-17 | |
| | | | **TECHNICAL FIELDS SEARCHED (Int. Cl.4)** |

The present search report has been drawn up for all claims

| Place of search | Date of completion of the search | Examiner |
|---|---|---|
| VIENNA | 09-12-1987 | WOLF |